# EUROPEAN PATENT APPLICATION

(11) **EP 2 210 615 A1**
(43) Date of publication of application: **28.07.2010**
(21) Application number: 09382006.6
(22) Date of filing: 21.01.2009
(51) Int. Cl.: A61K 45/06, A61K 31/519, A61K 31/44, A61P 29/00

(54) **Combinations comprising methotrexate and DHODH inhibitors**

(71) Applicant: Almirall, S.A., 08022 Barcelona (ES)
(72) Inventor: Godessart Marina, Nuria, 08028, Barcelona (ES); Pizcueta Lalanza, Maria Pilar, 08017, Barcelona (ES)
(74) Representative: Elzaburu Marquez, Alberto

(57) **Abstract**

The present invention provides a combination which comprises (a) methotrexate and (b) a non-hepatotoxic DHODH inhibitor of formula (I): 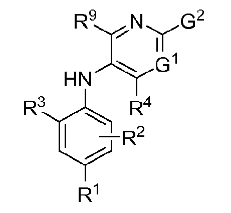 wherein:
R¹ is selected from the group consisting ofhydrogen atoms, halogen atoms, C₁₋₄ alkyl, C₃₋₄ cycloalkyl, -CF₃ and -OCF₃,
R² is selected from the group consisting of hydrogen atoms, halogen atoms and C₁₋₄ alkyl groups,
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ represents a group selected from N and CR⁶ wherein R⁶ is selected from the group consisting ofhydrogen atoms, halogen atoms, C₁₋₄ alkyl, C₃₋₄ cycloalkyl, C₁₋₄ alkoxy, - CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G² represents a group selected from:
• a hydrogen atom, a hydroxy group, a halogen atom, a C₃₋₄ cycloalkyl group, a C₁₋₄ alkoxy group and NR^{a}R^{b}, wherein
R^{a} represents a C₁₋₄ alkyl group and R^{b} is selected from a group consisting of C₁₋₄ alkyl group and C₁₋₄ alkoxy-C₁₋₄ alkyl group, or
R^{a} and R^{b} together with the nitrogen atom to which they are attached form a saturated 6 to 8 membered heterocyclic ring optionally containing one oxygen atom as an additional heteroatom,
• a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 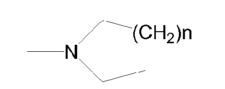 wherein n is an integer from 0 to 3,
and
• a phenyl group which is optionally substituted by one or more substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula

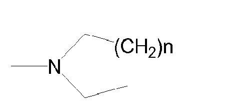

wherein n is an integer from 0 to 3
or, when G' represents CR⁶, G² together with R⁶ forms a non-aromatic C₅₋₁₀ carbocyclic group or a C₆₋₁₀ aryl group,

and the pharmaceutically acceptable salts and N-oxides thereof.

## Description

The present invention relates to new combinations of methotrexate with DHODH inhibitors. These combinations are useful in the treatment, prevention or suppression of diseases and disorders known to be susceptible to improvement with methotrexate and / or by inhibition of dihydroorotate dehydrogenase, such as autoimmune diseases, immune and inflammatory diseases, destructive bone disorders, malignant neoplastic diseases, angiogenic-related disorders, viral diseases, and infectious diseases.

### BACKGROUND OF THE INVENTION

Methotrexate (MTX) is an antimetabolite and immunomodulator that affects many intracellular pathways of purine metabolism. It is effective in reducing the signs and symptoms of rheumatoid arthritis (RA), as well as in slowing or halting radiographic damage. Due to its efficacy, case of administration and relatively low cost, MTX has become the first-line oral therapy in most patients with RA. In those patients who have an incomplete response to MTX, another DMARD (disease modifying anti-rheumatic drug) is added on top of it. Thus, combination therapy with MTX is more and more frequent in the clinical practice.

Leflunomide is an example of such a DMARD. It was approved in September 1998 for use in RA. It has been shown to reduce the signs and symptoms of the disease, to inhibit structural damage (evidenced by X-ray erosions and joint space narrowing) and to improve physical function. Teriflunomide is the active metabolite of Leflunomide.

Methotrexate is thought to act primarily on purine pathways of cellular metabolism, whereas Leflunomide affects pyrimidine pathways. Given the diverse intracellular pathways affected by both drugs, the combination of Leflunomide and methotrexate has the potential for biochemical synergy. In fact, it has been reported that the combination of both agents led to considerable clinical improvement (see for example, Kremer JM et al. "Concomitant Leflunomide therapy in patients with active rheumatoid arthritis despite stable doses of methotrexate". Ann. Intern. Med., 2002; 137, 726-733).

Unfortunately, both methotrexate and leflunomide have serious adverse effects, in particular hepatotoxicity. Methotrexate may cause fatal liver damage such as fibrosis and cirrhosis after prolonged use. Liver enzyme increases are frequently seen during treatment with methotrexate. Hence, regular and careful monitoring of patients taking MTX is essential, particularly when MTX is combined with other DMARDs.

The most common reported adverse events of Leflunomide include diarrhoea, dyspepsia, rash, hair loss, hypertension and elevated hepatic enzymes. The hepatotoxicity potential is of special relevance and regular laboratory tests, including blood tests of liver function, must be performed for all patients taking this medication. Leflunomide is not recommended for use in patients with evidence of hepatitis B or C infection or significant hepatic impairment.

Clinical trials have reported that the number of patients experiencing an increase in liver markers (measured as transaminase levels) is notably higher in the group of Leflunomide plus MTX than in the group of MTX alone. The product information for Leflunomide warns against combination with methotrexate on the basis that such combination therapy can lead to additive or even synergistic hepatotoxicity.

The adverse hepatic effects of leflunomide, and in particular of its active metabolite, teriflunomide, have been attributed to its activity as inhibitor of dihydroorotate dehydrogenase (DHODH). Liver toxicity has thus been identified as an adverse effect directly derived from the mechanism of action of DHODH-inhibitors, which has hampered the development of this class of compounds.

### DESCRIPTION OF THE INVENTION

It has now been found that, contrary to general belief, inhibition of DHODH is not responsible for the liver damage produced by leflunomide and that certain non-hepatotoxic DHODH inhibitors are particularly suitable for combination with methotrexate.

It is known that inhibition of DHODH produces immunosuppressant and antiproliferative effects. DHODH inhibitors can therefore be used as immunosuppressants and as antiproliferatives in the treatment of autoimmune, inflammatory and proliferative diseases, like RA.

The present invention is based on the surprising finding that the inhibition of DHODH is not linked to hepatotoxicity and, consequently, DHODH inhibitors devoid ofhepatotoxic potential represent an important contribution to the treatment of these diseases, due to their advantageous combinability with MTX, the most commonly used first-line drug in RA treatment.

We have developed an *in vivo* model of hepatotoxicity assessment in mice, in which test compounds are administered by intraperitoneal route to maximise liver exposure. In this model, Teriflunomide, the active metabolite of Leflunomide, has shown a drastic increase in the levels of transaminases and bilirrubin in plasma, whereas DHODH inhibitors do not show an increase in any of the plasma liver markers in the same model, while maintaining their efficacy in arthritis.

Thus, the present invention is directed to a combination which comprises (a) methotrexate and (b) a non-hepatotoxic DHODH inhibitor of formula (I): 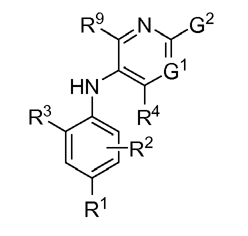 wherein:
R¹ is selected from the group consisting of hydrogen atoms, halogen atoms, C₁₋₄ alkyl, C₃₋₄ cycloalkyl, -CF₃ and -OCF₃,
R² is selected from the group consisting of hydrogen atoms, halogen atoms and C₁₋₄ alkyl groups,
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ represents a group selected from N and CR⁶ wherein R⁶ is selected from the group consisting of hydrogen atoms, halogen atoms, C₁₋₄ alkyl, C₃₋₄ cycloalkyl, C₁₋₄ alkoxy, - CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G² represents a group selected from:
   - a hydrogen atom, a hydroxy group, a halogen atom, a C₃₋₄ cycloalkyl group, a C₁₋₄ alkoxy group and -NR^{a}R^{b}, wherein
      R^{a} represents a C₁₋₄ alkyl group and R^{b} is selected from a group consisting of C₁₋₄ alkyl group and C₁₋₄ alkoxy-C₁₋₄ alkyl group, or
      R^{a} and R^{b} together with the nitrogen atom to which they are attached form a saturated 6 to 8 membered heterocyclic ring optionally containing one oxygen atom as an additional heteroatom,
   - a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 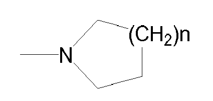 wherein n is an integer from 0 to 3,
      and
   - a phenyl group which is optionally substituted by one or more substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 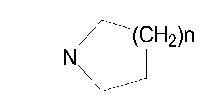
   wherein n is an integer from 0 to 3
   or, when G' represents CR⁶, G² together with R⁶ forms a non-aromatic C₅₋₁₀ carbocyclic group or a C₆₋₁₀ aryl group,
and the pharmaceutically acceptable salts and N-oxides thereof.

As used herein the term alkyl embraces optionally substituted, linear or branched hydrocarbon radicals having 1 to 4 carbon atoms. Preferred substiuents on the alkyl groups are halogen atoms and hydroxy groups.

Examples include methyl, ethyl, *n*-propyl, *i*-propyl, *n*-butyl, *sec*-butyl and *tert*-butyl radicals.

As used herein the term alkoxy embraces optionally substituted, linear or branched oxygen containing radicals each having 1 to 4 carbon atoms. Preferred substiuents on the alkoxy groups are halogen atoms and hydroxy groups.

Examples include methoxy, ethoxy, *n*-propoxy, *i*-propoxy, *n*-butoxy, *sec*-butoxy and *tert-*butoxy radicals.

As used herein, the term cycloalkyl embraces optionally substituted saturated carbocyclic radicals and, unless otherwise specified, a cycloalkyl radical typically has from 3 to 7 carbon atoms, preferably from 3 to 4 carbon atoms.

Examples include cyclopropyl, cyclobutyl, cyclopentyl, cyclohexyl and cycloheptyl. When a cycloalkyl radical carries 2 or more substituents, the substituents may be the same or different. Preferred substiuents on the cycloalkyl groups are halogen atoms and hydroxy groups.

As used herein, the term cycloalkoxy embraces saturated oxy-containing carbocyclic radicals and, unless otherwise specified, a cycloalkoxy radical typically has from 3 to 8 carbon atoms, preferably from 3 to 4 carbon atoms.

Examples include cyclopropyloxy, cyclobutyloxy, cyclopentyloxy, cyclohexyloxy and cycloheptyloxy. When a cycloalkoxy radical carries 2 or more substituents, the substituents may be the same or different. Preferred substiuents on the cycloalkoxy groups are halogen atoms and hydroxy groups.

As used herein, the term aryl radical embraces typically optionally substituent C₆-C₁₀ monocyclic or polycyclic aryl radical such as phenyl, naphthyl, anthranyl and phenanthryl. Phenyl is preferred.

A said optionally substituted aryl radical is typically unsubstituted or substituted with 1, 2 or 3 substituents which may be the same or different. The substituents are preferably selected from halogen atoms, preferably fluorine atoms, hydroxy groups, alkoxycarbonyl groups in which the alkyl moiety has from 1 to 4 carbon atoms, hydroxycarbonyl groups, carbamoyl groups, nitro groups, cyano groups, C₁-C₄ alkyl groups, C₁-C₄ alkoxy groups and C₁-C₄ hydroxyalkyl groups. When an aryl radical carries 2 or more substituents, the substituents may be the same or different. Unless otherwise specified, the substituents on an aryl group are typically themselves unsubstituted.

As used herein, the terms heteroaryl and heteroaromatic ring are used interchangeably and are typically 5- to 14- membered ring systems, preferably 5- to 10- membered ring systems, comprising at least one heteroaromatic ring and containing at least one heteroatom selected from O, S and N. A heteroaryl radical may be a single ring (monocyclic) or two or more fused rings (polycyclic) wherein at least one ring contains a heteroatom.

As used herein, the term heterocyclyl radical is typically a non-aromatic, saturated or unsaturated C₃-C₁₀ carbocyclic ring system, such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclyl radicals are preferred.

As used herein, the term halogen atom refers typically to chlorine, fluorine, bromine and iodine atoms, preferably fluorine, chlorine and bromine atoms. The term halo when used as a prefix has the same meaning.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or *p*-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge on the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

In the particular case where R³ is a COOH group, it is advantageous to have salts derived from the corresponding carboxylic acid by replacement of the hydrogen atom of the carboxylic group with a cation derived from a pharmaceutically acceptable base as described above.

As used herein, an N-oxide is formed from the tertiary basic amines or imines present in the molecule, using a convenient oxidising agent.

Typically, R¹ is selected from the group consisting of hydrogen atoms, fluorine atoms, chlorine atoms, bromine atoms, C₁₋₄ alkyl, C₃₋₄ cycloalkyl and -CF₃ groups.

Typically R² is selected from the group consisting of hydrogen atoms, halogen atoms and methyl groups.

Typically, G¹ is selected from the group consisting of nitrogen atoms, CCI, CF, CH, C(CH₃), C(cyclopropyl), C(phenyl) and C(CF₃) groups.

Typically G² represents a group selected from:
- a hydrogen atom, a halogen atom, a C₃₋₄ cycloalkyl group, a C₁₋₂ alkoxy group and -NR^{a}R^{b}, wherein
   R^{a} represents a C₁₋₂ alkyl group and R^{b} is selected from the group consisting of C₁₋₂ alkyl groups and C₁₋₂ alkoxy-C₁₋₂ alkyl groups, or
   R^{a} and R^{b} together with the nitrogen atom to which they are attached form a saturated 6 or 7 membered heterocyclic ring optionally containing one oxygen atom as an additional heteroatom,
- a monocyclic or bicyclic 5 to 10 membered heteroaromatic ring containing one or two nitrogen atoms which is optionally substituted by one or more substituents selected from halogen atoms and C₁₋₄ alkyl groups,
and
- a phenyl group which is optionally substituted by one, two or three substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸ and oxadiazolyl groups, which oxadiazolyl groups are optionally substituted by a C₁₋₄ alkyl or a C₃₋₇ cycloalkyl group and wherein R⁷ and R⁸ are independently selected from hydrogen atoms, linear or branched C₁₋₄ alkyl groups, C₃₋₄ cycloalkyl groups, or R⁷ and R⁸ together with the nitrogen atom to which they are attached form a group of formula 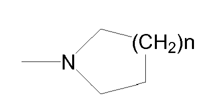 wherein n is 1 or 2,
   or, when G' represents CR⁶, G² together with R⁶ forms a non-aromatic C₆ carbocyclic group or a phenyl group.

More typically G² represents a group selected from:
- a hydrogen atom, a fluorine atom, a cyclopropyl group, a methoxy group, -NMeEt, -NEt₂, -N(Me)-(CH₂)₂-O-CH₃, 6-morpholinyl, azepan-1-yl and piperidin-1-yl,
- a pyridinyl, pyrimidinyl, quinolinyl or pyrazinyl ring optionally substituted with one or two substituents selected from Me and F
   and
- a phenyl group which is optionally substituted by one, two or three substituents selected from fluorine, chlorine, methyl, hydroxyl, methoxy, ethoxy, isopropyloxy, cyclopropyl, cyclopropyloxy, cyano, -CF₃, -OCF₃ ,oxadiazolyl and -CONR⁷R⁸ groups, which oxadiazolyl groups are optionally substituted by a methyl group and wherein R⁷ and R⁸ are independently selected from hydrogen atom, methyl group, isopropyl group, cyclopropyl group, or R⁷ and R⁸ together with the nitrogen atom to which they are attached form a group of formula 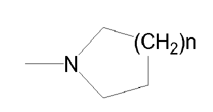 wherein n is 1,
   or, when G' represents CR⁶, G² together with R⁶ forms a non-aromatic C₆ carbocyclic group or a phenyl group.

Even more typically, G² represents a group selected from methoxy group, cyclopropyl group and optionally substituted phenyl, pyridyl, quinolynyl, pyrimidinyl and pyrazinyl groups.

In one embodiment of the present invention, R⁹ represents a hydrogen atom, and G² represents a group selected from:
- a monocyclic or bicyclic 5 to 10 membered heteroaromatic ring containing a nitrogen atom which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, lineal or branched C₁₋₄ alkyl group, C₃₋₇ cycloalkyl group, or R⁷ and R⁸ together with the nitrogen atom to which they are attached form a group of formula 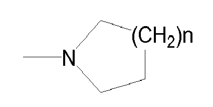 wherein n is an integer from 0 to 3,
   and
- a phenyl group which is optionally substituted by one or more substituents selected from halogen atoms, C₁₋₄ alkyl, C₁₋₄ alkoxy, C₃₋₄cycloalkyl, C₃₋₄ cycloalkoxy, -CF₃, -OCF₃, -CONR⁷R⁸, oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R⁷ and R⁸ are independently selected from hydrogen atom, lineal or branched C₁₋₄ alkyl group, a C₃₋₇ cycloalkyl group, or R⁷ and R⁸ together with the nitrogen atom to which they are attached form a group of formula 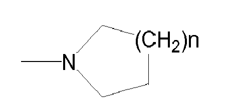 wherein n is an integer from 0 to 3
   and the pharmaceutically acceptable salts and N-oxides thereof.

Typically, R¹ is selected from the group consisting of C₁₋₄ alkyl, C₃₋₄ cycloalkyl and -CF₃, preferably methyl and cyclopropyl group, more preferably a cyclopropyl group.

Typically, R² is selected from a hydrogen or halogen atom, preferably a hydrogen atom.

Typically, R³ is selected from COOR⁵, -CONHR⁵ and tetrazolyl group; preferably R³ is a COOH group.

Typically, R⁴ represents a hydrogen atom or a methyl group, preferably a hydrogen atom.

Typically, R⁹ represents a hydrogen atom.

Typically, G¹ represents a group selected from N, CH, C(CH₃), C(cyclopropyl), C(phenyl) or C(CF₃) groups.

Typically, G² is selected from the group consisting of a methoxy group, a cyclopropyl group and optionally substituted phenyl, pyridyl, quinolynyl, pyrimidinyl and pyrazinyl groups, more preferably, G² is selected from the group consisting of optionally substituted phenyl, pyridyl, quinolynyl, pyrimidinyl and pyrazinyl groups, most preferably selected from optionally substituted phenyl, 4-pyridyl, 5-quinolynyl and 2-pyrazinyl groups.

In yet another embodiment of the present invention, R¹ is selected from a methyl or cyclopropyl group, R² represents a hydrogen atom, R³ is a COOH group, R⁴ represents a hydrogen atom or a methyl group, G¹ is selected from N, CH, C(CH₃), C(cyclopropyl), C(phenyl) and C(CF₃) groups, and G² represents a group selected from the group consisting of optionally substituted phenyl, 4-pyridyl, 5-quinolynyl and 2-pyrazinyl groups, more preferably R⁹ represent a hydrogen group.

In yet another embodiment of the present invention, R¹ is selected from a methyl or cyclopropyl group, R² represents a hydrogen atom, R³ is a COOH group, R⁴ represents a hydrogen atom, G¹ is selected from nitrogen atoms and CH, C(CH₃) and C(CF₃) groups and G² represents a phenyl group optionally substituted with one or two substituents selected from chloro, fluoro, methoxy, ethoxy, isopropoxy, trifluoromethoxy and -CONR⁷R⁸, wherein R⁷ is hydrogen and R⁸ is cyclopropyl or R⁷ and R⁸ together with the nitrogen atom to which they are attached form a group of formula 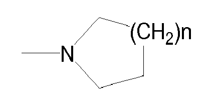 wherein n is 1.

Preferably, the DHODH inhibitor is one of:
1. 5-cyclopropyl-2-(2-phenylpyrimidin-5-ylamino)benzoic acid
2. 2-(6-Cyclopropyl-5-methylpyridin-3-ylamino)-5-methylbenzoic acid
3. 5-(2-Carboxy-4-cyclopropylphenylamino)-3-methyl-2-phenylpyridine 1-oxide
4. 5-Methyl-2-(6-(3-(trifluoromethyl)phenyl)pyridin-3-ylamino)benzoic acid
5. 5-cyclopropyl-2-(6-hydroxy-5-phenylpyridin-3-ylamino)benzoic acid
6. 5-cyclopropyl-2-(2-(2,6-difluoro-4-hydroxyphenyl)pyrimidin-5-ylamino)benzoic acid
7. 5-Cyclopropyl-2-(6-methoxy-5-phenylpyridin-3-ylamino) benzoic acid
8. 2-(5-Fluoro-6-phenylpyridin-3-ylamino)-5-methylbenzoic acid
9. 2-(6-(Ethyl(methyl)amino)-5-methylpyridin-3-ylamino)-5-methylbenzoic acid
10. 5-Cyclopropyl-2-(3'-fluoro-2,4'-bipyridin-5-ylamino)benzoic acid
11. 2-(6-(Diethylamino)-5-methylpyridin-3-ylamino)-5-methylbenzoic acid
12. 2-(6-((2-Methoxyethyl)(methyl)amino)-5-methylpyridin-3-ylamino)-5-methylbenzoic acid
13. 2-(5-Chloro-6-phenylpyridin-3-ylamino)-5-methylbenzoic acid
14. 5-Cyclopropyl-2-(2-(2-cyclopropylphenyl)pyrimidin-5-ylamino)benzoic acid
15. 5-cyclopropyl-2-(5-phenylpyridin-3-ylamino) benzoic acid
16. 5-methyl-2-(quinolin-3-ylamino)benzoic acid
17. 5-methyl-2-(5,6,7,8-tetrahydroquinolin-3-ylamino)benzoic acid
18. 2-(5-Chloro-2-phenylpyridin-3-ylamino)-5-methylbenzoic acid
19. 5-Cyclopropyl-2-(5,6-diphenylpyridin-3-ylamino)benzoic acid
20. 5-cyclopropyl-2-(2-(2,6-difluorophenyl)pyrimidin-5-ylamino)benzoic acid
21. 5-Cyclopropyl-2-(5-methylpyridin-3-ylamino) benzoic acid
22. 2-(2-(3-Cyclopropoxyphenyl)pyrimidin-5-ylamino)-5-cyclopropylbenzoic acid
23. 5-Methyl-2-(6-morpholinopyridin-3-ylamino)benzoic acid
24. 5-Methyl-2-(5-methyl-6-morpholinopyridin-3-ylamino)benzoic acid
25. 5-cyclopropyl-2-(6-cyclopropyl-5-phenylpyridin-3-ylamino) benzoic acid
26. 2-(6-(2-Cyclopropylphenyl)-5-methylpyridin-3-ylamino)-5-methylbenzoic acid
27. 2-(6-(2-Cyanophenyl)-5-methylpyridin-3-ylamino)-5-methylbenzoic acid
28. 2-(2-(3-Chlorophenyl)pyrimidin-5-ylamino)-5-cyclopropylbenzoic acid
29. 5-Methyl-2-(6-phenyl-5-(trifluoromethyl)pyridin-3-ylamino)benzoic acid
30. 5-Methyl-2-(5-methyl-6-(piperidin-1-yl)pyridin-3-ylamino)benzoic acid
31. 2-(6-(Azepan-1-yl)-5-methylpyridin-3-ylamino)-5-methylbenzoic acid
32. 2-(6-(3-Methoxyphenyl)-5-phenylpyridin-3-ylamino)-5-methylbenzoic acid
33. 2-(2,3'-bipyridin-5-ylamino)-5-cyclopropylbenzoic acid
34. 2-(3'-chloro-2,4'-bipyridin-5-ylamino)-5-methylbenzoic acid
35. 5-Methyl-2-(3-methyl-2,2'-bipyridin-5-ylamino)benzoic acid
36. 2-(5,6-Difluoropyridin-3-ylamino)-5-methylbenzoic acid
37. 2-(6-(3-Methoxyphenyl)pyridin-3-ylamino)benzoic acid
38. 2-(6-(3-Ethoxyphenyl)pyridin-3-ylamino)benzoic acid
39. 2-(6-(3-Ethoxyphenyl)pyridin-3-ylamino)-5-fluorobenzoic acid
40. 2-(6-(3-Ethoxyphenyl)-5-methylpyridin-3-ylamino)benzoic acid
41. 2-(6-(3-Ethoxyphenyl)pyridin-3-ylamino)-5-methylbenzoic acid
42. 2-(6-(3-Ethoxyphenyl)-5-methylpyridin-3-ylamino)-5-methylbenzoic acid
43. 2-(6-(3-Ethoxy-2-fluorophenyl)pyridin-3-ylamino)benzoic acid
44. 2-(6-(3-Ethoxyphenyl)-4-methylpyridin-3-ylamino)-5-methylbenzoic acid
45. 2-(6-(3-Ethoxyphenyl)-4-methylpyridin-3-ylamino)benzoic acid
46. 5-Bromo-2-(6-(3-ethoxyphenyl)pyridin-3-ylamino)benzoic acid
47. 5-Chloro-2-(6-(3-ethoxyphenyl)pyridin-3-ylamino)benzoic acid
48. 2-(6-(5-Ethoxy-2-fluorophenyl)pyridin-3-ylamino)benzoic acid
49. 2-(6-(3-Ethoxyphenyl)-5-methylpyridin-3-ylamino)-5-(trifluoromethyl)benzoic acid
50. 2-(6-(3-Methoxyphenyl)-5-methylpyridin-3-ylamino)-5-(trifluoromethyl)benzoic acid
51. 2-(6-(3-Methoxyphenyl)-5-methylpyridin-3-ylamino)-5-methylbenzoic acid
52. 2-(6-(3-Methoxyphenyl)-5-methylpyridin-3-ylamino)-6-methylbenzoic acid
53. 5-Fluoro-2-(6-(3-methoxyphenyl)-5-methylpyridin-3-ylamino)benzoic acid
54. 2-(6-(5-Ethoxy-2-fluorophenyl)pyridin-3-ylamino)-5-methylbenzoic acid
55. 2-(6-(2-Fluoro-5-methoxyphenyl)-5-methylpyridin-3-ylamino)-5-methylbenzoic acid
56. Ethyl 2-(6-(2-fluoro-5-methoxyphenyl)-5-methylpyridin-3-ylamino)-5-methylbenzoate
57. 2-(6-(2-Fluorophenyl)pyridin-3-ylamino)-5-methylbenzoic acid
58. 2-(6-(3-Methoxyphenyl)-5-phenylpyridin-3-ylamino)-5-methylbenzoic acid
59. Ethyl 2-(6-(3-methoxyphenyl)-5-phenylpyridin-3-ylamino)-5-methylbenzoate
60. 5-Methyl-2-(5-methyl-6-phenylpyridin-3-ylamino)benzoic acid
61. Ethyl 5-methyl-2-(5-methyl-6-phenylpyridin-3-ylamino)benzoate
62. 5-Methyl-2-(5-methyl-6-(3-(trifluoromethoxy)phenyl)pyridin-3-ylamino)benzoic acid
63. Ethyl 5-methyl-2-(5-methyl-6-(3-(trifluoromethoxy)phenyl)pyridin-3-ylamino)benzoate
64. 2-(5-Cyclopropyl-6-(3-methoxyphenyl)pyridin-3-ylamino)-5-methylbenzoic acid
65. Ethyl 2-(5-cyclopropyl-6-(3-methoxyphenyl)pyridin-3-ylamino)-5-methylbenzoate
66. 2-(6-(2-Fluoro-5-isopropoxyphenyl)pyridin-3-ylamino)-5-methylbenzoic acid
67. 2-(6-(3-Isopropoxyphenyl)-5-methylpyridin-3-ylamino)-5-methylbenzoic acid
68. Ethyl 2-(6-(3-isopropoxyphenyl)-5-methylpyridin-3-ylamino)-5-methylbenzoate
69. 2-(6-(3-Cyclopropoxyphenyl)-5-methylpyridin-3-ylamino)-5-methylbenzoic acid
70. *tert*-Butyl 2-(6-(3-cyclopropoxyphenyl)-5-methylpyridin-3-ylamino)-5-methylbenzoate
71. 2-(6-(2-Chlorophenyl)-5-methylpyridin-3-ylamino)-5-methylbenzoic acid
72. *tert*-Butyl 2-(6-(2-chlorophenyl)-5-methylpyridin-3-ylamino)-5-methylbenzoate
73. 2-(6-(3-Carbamoylphenyl)-5-methylpyridin-3-ylamino)-5-methylbenzoic acid
74. Ethyl 2-(6-(3-carbamoylphenyl)-5-methylpyridin-3-ylamino)-5-methylbenzoate
75. 2-(6-(2-Fluoro-5-methoxyphenyl)-4-methylpyridin-3-ylamino)-5-methylbenzoic acid
76. Ethyl 2-(6-(2-fluoro-5-methoxyphenyl)-4-methylpyridin-3-ylamino)-5-methylbenzoate
77. 2-(6-(3-Methoxyphenyl)-5-(trifluoromethyl)pyridin-3-ylamino)-5-methylbenzoic acid
78. Ethyl 2-(6-(3-methoxyphenyl)-5-(trifluoromethyl)pyridin-3-ylamino)-5-methylbenzoate
79. 2-(6-(3-(Dimethylcarbamoyl)phenyl)-5-methylpyridin-3-ylamino)-5-methylbenzoic acid
80. Ethyl 2-(6-(3-(dimethylcarbamoyl)phenyl)-5-methylpyridin-3-ylamino)-5-methylbenzoate
81. 2-(6-(3-Isopropoxyphenyl)-5-methylpyridin-3-ylamino)-3-methylbenzoic acid
82. *tert*-Butyl 2-(6-(3-isopropoxyphenyl)-5-methylpyridin-3-ylamino)-3-methylbenzoate
83. 3-Methyl-2-(5-methyl-6-phenylpyridin-3-ylamino)benzoic acid
84. *tert*-Butyl 3-methyl-2-(5-methyl-6-phenylpyridin-3-ylamino)benzoate
85. 2-(6-(2-Chlorophenyl)pyridin-3-ylamino)-5-methylbenzoic acid
86. *tert*-Butyl 2-(6-(2-chlorophenyl)pyridin-3-ylamino)-5-methylbenzoate
87. 3-Fluoro-2-(6-(3-methoxyphenyl)-5-methylpyridin-3-ylamino)benzoic acid
*88. tert-Butyl* 3-fluoro-2-(6-(3-methoxyphenyl)-5-methylpyridin-3-ylamino) benzoate
89. 5-Cyclopropyl-2-(5-methyl-6-(3-(trifluoromethoxy)phenyl)pyridin-3-ylamino)benzoic acid
90. Ethyl 5-cyclopropyl-2-(5-methyl-6-(3-(trifluoromethoxy)phenyl)pyridin-3-ylamino)benzoate
91. 5-Cyclopropyl-2-(5-methyl-6-phenylpyridin-3-ylamino)benzoic acid
92. Ethyl 5-cyclopropyl-2-(5-methyl-6-phenylpyridin-3-ylamino)benzoate
93. 5-Methyl-2-(5-methyl-6-(2-(trifluoromethyl)phenyl)pyridin-3-ylamino)benzoic acid
94. *tert-Butyl* 5-methyl-2-(5-methyl-6-(2-(trifluoromethyl)phenyl)pyridin-3-ylamino)benzoate
95. 2-(6-(3-Chlorophenyl)-5-methylpyridin-3-ylamino)-5-methylbenzoic acid
96. *tert*-Butyl 2-(6-(3-chlorophenyl)-5-methylpyridin-3-ylamino)-5-methylbenzoate
97. 2-(6-(2-Fluorophenyl)-5-methylpyridin-3-ylamino)-5-methylbenzoic acid
98. *tert*-Butyl 2-(6-(2-fluorophenyl)-5-methylpyridin-3-ylamino)-5-methylbenzoate
99. 5-Methyl-2-(5-methyl-6-(quinolin-5-yl)pyridin-3-ylamino)benzoic acid
100. *tert*-Butyl 5-methyl-2-(5-methyl-6-(quinolin-5-yl)pyridin-3-ylamino)benzoate
101. 2-(3'-Fluoro-3-methyl-2,4'-bipyridin-5-ylamino)-5-methylbenzoic acid
102. *tert*-Butyl 2-(3'-fluoro-3-methyl-2,4'-bipyridin-5-ylamino)-5-methylbenzoate
103. 5-Methyl-2-(5-methyl-6-(pyrazin-2-yl)pyridin-3-ylamino)benzoic acid
104. *tert*-Butyl 5-methyl-2-(5-methyl-6-(pyrazin-2-yl)pyridin-3-ylamino)benzoate
105. 5-Cyclopropyl-2-(6-phenyl-5-(trifluoromethyl)pyridin-3-ylamino)benzoic acid
106. Ethyl 5-cyclopropyl-2-(6-phenyl-5-(trifluoromethyl)pyridin-3-ylamino)benzoate
107. 5-Cyclopropyl-2-(6-(3-methoxyphenyl)-5-(trifluoromethyl)pyridin-3-ylamino)benzoic acid
108. Ethyl 5-cyclopropyl-2-(6-(3-methoxyphenyl)-5-(trifluoromethyl)pyridin-3-ylamino)benzoate
109. 5-Chloro-2-(6-(2-fluorophenyl)pyridin-3-ylamino)benzoic acid
110. 5-Chloro-2-(6-(2-chlorophenyl)pyridin-3-ylamino)benzoic acid
111. 5-Chloro-2-(6-(quinolin-5-yl)pyridin-3-ylamino)benzoic acid
112. 2-(6-(2-Chlorophenyl)pyridin-3-ylamino)-5-cyclopropylbenzoic acid
113. Ethyl 2-(6-(2-chlorophenyl)pyridin-3-ylamino)-5-cyclopropylbenzoate
114. 5-Chloro-2-(6-(2-(trifluoromethyl)phenyl)pyridin-3-ylamino)benzoic acid
115. 5-Fluoro-2-(6-(2-(trifluoromethyl)phenyl)pyridin-3-ylamino)benzoic acid
116. 2-(3'-Fluoro-2,4'-bipyridin-5-ylamino)-5-methylbenzoic acid
117. 2-(2-(2-Fluorophenyl)pyrimidin-5-ylamino)-5-methylbenzoic acid
118. tert-Butyl 2-(2-(2-fluorophenyl)pyrimidin-5-ylamino)-5-methylbenzoate
119. 2-(6-(2,6-Difluorophenyl)pyridin-3-ylamino)-5-methylbenzoic acid
120. Ethyl 2-(6-(2,6-difluorophenyl)pyridin-3-ylamino)-5-methylbenzoate
121. 2-(2-(2-Chlorophenyl)pyrimidin-5-ylamino)-5-cyclopropylbenzoic acid
122. Methyl 2-(2-(2-chlorophenyl)pyrimidin-5-ylamino)-5-cyclopropylbenzoate
123. 2-(2-(2-Chlorophenyl)pyrimidin-5-ylamino)-5-methylbenzoic acid
124. *tert*-Butyl 2-(2-(2-chlorophenyl)pyrimidin-5-ylamino)-5-methylbenzoate
125. 5-Methyl-2-(5-methyl-6-(3-(pyrrolidine-1-carbonyl)phenyl)pyridin-3-ylamino)benzoic acid
126. 2-(6-(3-(Cyclopropylcarbamoyl)phenyl)-5-methylpyridin-3-ylamino)-5-methylbenzoic acid
127. 5-Cyclopropyl-2-(2-(2-fluorophenyl)pyrimidin-5-ylamino)benzoic acid
128. 2-(2-(2-trifluoromethylphenyl)pyrimidin-5-ylamino)-5-cyclopropylbenzoic acid
129. 2-(2-o-tolylpyrimidin-5-ylamino)-5-cyclopropylbenzoic acid
130. 2-(2-(2-cyclopropoxyphenyl)pyrimidin-5-ylamino)-5-cyclopropylbenzoic acid
131. 2-(2-(2,5-difluorophenyl)pyrimidin-5-ylamino)-5-cyclopropylbenzoic acid
132. 2-(2-(2,3-difluorophenyl)pyrimidin-5-ylamino)-5-cyclopropylbenzoic acid
133. 2-(2-(2-fluoro-5-chlorophenyl)pyrimidin-5-ylamino)-5-cyclopropylbenzoic acid
134. 2-(2-(2-trifluoromethylphenyl)pyrimidin-5-ylamino)-5-methylbenzoic acid
135. 2-(2-(2-fluoro-5-trifluoromethoxyphenyl)pyrimidin-5-ylamino)-5-cyclopropylbenzoic acid
136. 2-(6-(2-trifluoromethylphenyl)pyridin-3-ylamino)-5-methylbenzoic acid
137. 2-(6-phenylpyridin-3-ylamino)-5-cyclopropylbenzoic acid
138. 2-(6-(2-fluorophenyl)pyridin-3-ylamino)-5-cyclopropylbenzoic acid
139. 2-(6-(3,5-difluoropyridin-4-yl)pyridin-3-ylamino)-5-methylbenzoic acid
140. 2-(6-(3-cyclopropylcarbamoylphenyl)pyridin-3-ylamino)-5-methylbenzoic acid
141. 2-(6-(2,4-difluorophenyl)pyridin-3-ylamino)-5-methylbenzoic acid
142. 2-(6-(2,5-difluorophenyl)pyridin-3-ylamino)-5-methylbenzoic acid
143. 2-(6-(2-fluorophenyl)pyridin-3-ylamino)-5-cyclopropyl-3-fluorobenzoic acid
144. 2-(6-(2,3,6-trifluorophenyl)pyridin-3-ylamino)-5-methylbenzoic acid
145. 2-(6-(3-(5-methyl-1,3,4-oxadiazol-2-yl)phenyl)pyridin-3-ylamino)-5-methylbenzoic acid
146. 2-(5-methyl-6-(pyrimidin-5-yl)pyridin-3-ylamino)-5-methylbenzoic acid
147. 2-(6-(2,3-difluorophenyl)pyridin-3-ylamino)-5-methylbenzoic acid
148. 2-(6-(5-fluoro-2-methoxyphenyl)-5-methylpyridin-3-ylamino)-5-methylbenzoic acid, and
149. 2-(6-(4-carbamoylphenyl)-5-methylpyridin-3-ylamino)-5-methylbenzoic acid
   or a pharmaceutically acceptable salt or N-oxide thereof
   more preferably, the DHODH inhibitor is one of:
   5-cyclopropyl-2-(2-phenylpyrimidin-5-ylamino)benzoic acid
   5-(2-Carboxy-4-cyclopropylphenylamino)-3-methyl-2-phenylpyridine 1-oxide
   5-Methyl-2-(6-(3-(trifluoromethyl)phenyl)pyridin-3-ylamino)benzoic acid
   2-(5-Fluoro-6-phenylpyridin-3-ylamino)-5-methylbenzoic acid
   5-Cyclopropyl-2-(3'-fluoro-2,4'-bipyridin-5-ylamino)benzoic acid
   2-(5-Chloro-6-phenylpyridin-3-ylamino)-5-methylbenzoic acid
   5-Cyclopropyl-2-(2-(2-cyclopropylphenyl)pyrimidin-5-ylamino)benzoic acid
   5-cyclopropyl-2-(5-phenylpyridin-3-ylamino) benzoic acid
   2-(5-Chloro-2-phenylpyridin-3-ylamino)-5-methylbenzoic acid
   5-Cyclopropyl-2-(5,6-diphenylpyridin-3-ylamino)benzoic acid
   5-cyclopropyl-2-(2-(2,6-difluorophenyl)pyrimidin-5-ylamino)benzoic acid
   2-(2-(3-Cyclopropoxyphenyl)pyrimidin-5-ylamino)-5-cyclopropylbenzoic acid
   5-Methyl-2-(6-morpholinopyridin-3-ylamino)benzoic acid
   5-Methyl-2-(5-methyl-6-morpholinopyridin-3-ylamino)benzoic acid
   5-cyclopropyl-2-(6-cyclopropyl-5-phenylpyridin-3-ylamino) benzoic acid
   2-(6-(2-Cyclopropylphenyl)-5-methylpyridin-3-ylamino)-5-methylbenzoic acid
   2-(6-(2-Cyanophenyl)-5-methylpyridin-3-ylamino)-5-methylbenzoic acid
   2-(2-(3-Chlorophenyl)pyrimidin-5-ylamino)-5-cyclopropylbenzoic acid
   5-Methyl-2-(6-phenyl-5-(trifluoromethyl)pyridin-3-ylamino)benzoic acid
   5-Methyl-2-(5-methyl-6-(piperidin-1-yl)pyridin-3-ylamino)benzoic acid
   2-(6-(Azepan-1-yl)-5-methylpyridin-3-ylamino)-5-methylbenzoic acid
   2-(2,3'-bipyridin-5-ylamino)-5-cyclopropylbenzoic acid
   2-(3'-chloro-2,4'-bipyridin-5-ylamino)-5-methylbenzoic acid
   5-Methyl-2-(3-methyl-2,2'-bipyridin-5-ylamino)benzoic acid
   2-(6-(3-Ethoxyphenyl)-5-methylpyridin-3-ylamino)-5-methylbenzoic acid
   2-(6-(3-Methoxyphenyl)-5-methylpyridin-3-ylamino)-5-methylbenzoic acid
   5-Methyl-2-(5-methyl-6-phenylpyridin-3-ylamino)benzoic acid
   2-(6-(3-Isopropoxyphenyl)-5-methylpyridin-3-ylamino)-5-methylbenzoic acid
   5-Cyclopropyl-2-(5-methyl-6-(3-(trifluoromethoxy)phenyl)pyridin-3-ylamino)benzoic acid
   5-Cyclopropyl-2-(5-methyl-6-phenylpyridin-3-ylamino)benzoic acid
   2-(6-(2-Fluorophenyl)-5-methylpyridin-3-ylamino)-5-methylbenzoic acid
   5-Cyclopropyl-2-(6-phenyl-5-(trifluoromethyl)pyridin-3-ylamino)benzoic acid
   5-Cyclopropyl-2-(6-(3-methoxyphenyl)-5-(trifluoromethyl)pyridin-3-ylamino)benzoic acid
   2-(6-(2-Chlorophenyl)pyridin-3-ylamino)-5-cyclopropylbenzoic acid
   2-(2-(2-Chlorophenyl)pyrimidin-5-ylamino)-5-cyclopropylbenzoic acid
   2-(2-(2-Chlorophenyl)pyrimidin-5-ylamino)-5-methylbenzoic acid
   2-(6-(2,6-Difluorophenyl)pyridin-3-ylamino)-5-methylbenzoic acid
   5-Methyl-2-(5-methyl-6-(3-(pyrrolidine-1-carbonyl)phenyl)pyridin-3-ylamino)benzoic acid
   2-(6-(3-(Cyclopropylcarbamoyl)phenyl)-5-methylpyridin-3-ylamino)-5-methylbenzoic acid,
   5-Cyclopropyl-2-(2-(2-fluorophenyl)pyrimidin-5-ylamino)benzoic acid.
      or a pharmaceutically acceptable salt or N-oxide thereof

Most preferably, the DHODH inhibitor is 5-Methyl-2-(6-(3-(trifluoromethyl)phenyl)pyridin-3-ylamino)benzoic acid, 5-cyclopropyl-2-(2-(2,6-difluorophenyl)pyrimidin-5-ylamino)benzoic acid, 2-(6-(2,6-Difluorophenyl)pyridin-3-ylamino)-5-methylbenzoic acid or 2-(6-(3-(Cyclopropylcarbamoyl)phenyl)-5-methylpyridin-3-ylamino)-5-methylbenzoic acid or a pharmaceutically acceptable salt or N-oxide thereof.

Preferably the active ingredients (a) and (b) form part of a single pharmaceutical composition.

Further provided is a combination as described above which further comprises (c) another compound selected from:
(i) Anti-TNF-alpha monoclonal antibodies such as Infliximab, Certolizumab pegol, Golimumab, Adalimumab and AME-527 from Applied Molecular Evolution
(ii) TNF-alpha Antagonists such as Etanercept, Lenercept, Onercept and Pegsunercept
(iii) Calcineurin (PP-2B) Inhibitors / INS Expression Inhibitors such as cyclosporine A, Tacrolimus and ISA-247 from Isotechnika
(iv) IL-1 Receptor Antagonists such as Anakinra and AMG-719 from Amgen
(v) Anti-CD20 monoclonal antibodies such as Rituximab, Ofatumumab, Ocrelizumab and Thru-015 from Trubion Pharmaceuticals
(vi) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745 and VX-702 (all from Vertex)
(vii) NF-kappaB (NFKB) Activation Inhibitors such as Sulfasalazine and Iguratimod
(viii) Another dihydrofolate reductase (DHFR) inhibitor such as Aminopterin and CH-1504 from Chelsea
(ix) Janus kinase (JAK) inhibitors, such as CP-690, 550 from Pfizer and INCB-18424, from Incyte.
(x) MEK inhibitor, such as ARRY-162 from Array
(xi) Sphingosine-1 phosphate receptor agonists, such as fingolimod (Novartis)

The present invention further provides use of (a) methotrexate and (b) a DHODH inhibitor of the invention for the preparation of a medicament for simultaneous, separate or sequential use in the treatment of a pathological condition or disease susceptible to amelioration by inhibition of dehydroorotate dehydrogenase.

Diseases or disorders in which DHODH inhibition plays a role include without limitation autoimmune diseases, immune and inflammatory diseases, destructive bone disorders, malignant neoplastic diseases, angiogenic-related disorders, viral diseases, and infectious diseases.

Autoimmune diseases which may be prevented or treated include but are not limited to rheumatoid arthritis, psoriatic arthritis, systemic lupus erythematosus, multiple sclerosis, psoriasis, ankylosing spondilytis, Wegener's granulomatosis, polyarticular juvenile idiopathic arthritis, inflammatory bowel disease such as ulcerative colitis and Crohn's disease, Reiter's syndrome, fibromyalgia and type-1 diabetes.

Immune and inflammatory diseases which may be prevented or treated include but are not limited to asthma, COPD, respiratory distress syndrome, acute or chronic pancreatitis, graft versus-host disease, chronic sarcoidosis, transplant rejection, contact dermatitis, atopic dermatitis, allergic rhinitis, allergic conjunctivitis, Behcet syndrome, inflammatory eye conditions such as conjunctivitis and uveitis.

Destructive bone disorders which may be prevented or treated include but are not limited to osteoporosis, osteoarthritis and multiple myeloma-related bone disorder.

Malignant neoplastic diseases that may be prevented or treated include but are not limited to prostate, ovarian and brain cancer.

Angiogenesis-related disorders that may be prevented or treated include but are not limited to haemangioma, ocular neovascularization, macular degeneration or diabetic retinopathy.

Viral diseases which may be prevented or treated include but are not limited to HIV infection, hepatitis and cytomegalovirus infection.

Infectious diseases which may be prevented or treated include but are not limited to sepsis, septic shock, endotoxic shock, Gram negative sepsis, toxic shock syndrome, Shigellosis and other protozoal infestations such as malaria.

Preferably, the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis. More preferably the pathological condition or disease is rheumatoid arthritis, psoriatic arthritis or psoriasis. Most preferably it is rheumatoid arthritis.

Also provided is a product comprising (a) methotrexate and (b) a DHODH inhibitor of the invention, as a combined preparation for simultaneous, separate or sequential use in the treatment of a human or animal patient suffering from or susceptible to a pathological condition or disease as defined above. Said product may optionally further comprise an active compound (c), as defined above.

Also provided is a kit of parts comprising (b) a DHODH inhibitor of the invention together with instructions for simultaneous, separate or sequential use in combination with (a) methotrexate, for the treatment of a human or animal patient suffering from or susceptible to a pathological condition or disease as defined above. Said kit may optionally further comprise an active compound (c), as defined above.

Also provided is a package comprising (b) a DHODH inhibitor of the invention and (a) methotrexate, for simultaneous, separate or sequential use in the treatment of a pathological condition or disease as defined above. Said package may optionally further comprise an active compound (c), as defined above.

Also provided is a use of (b) a DHODH inhibitor of the invention for the preparation of a medicament, for use in combination with (a) methotrexate, for the treatment of a pathological condition or disease as defined above.

Also provided is a use of (a) methotrexate, for the preparation of a medicament, for use in combination with (b) a DHODH inhibitor of the invention, for the treatment of a pathological condition or disease as defined above.

Also provided is a use as defined above wherein the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor.

Typically the medicament is for use in treating a human or animal patient suffering or susceptible to hepatic impairment or a condition that would be aggravated by hepatotoxicity. More typically, the said human or animal patient is suffering from liver fibrosis, hepatitis (typically hepatitis A to G), cirrhosis (typically caused by alcoholism) or liver cancer.

In one embodiment of the present invention, the combination, product, kit of parts or package comprises (b) a DHODH inhibitor of the invention, and (a) methotrexate, as the sole active components.

The fact that the DHODH inhibitors of the invention have reduced hepatic side effects is a finding of the invention. The present invention therefore also provides the use of a DHODH inhibitor of the invention, as defined above, in the manufacture of a medicament for use in treating or preventing a pathological condition or disease, as defined above, in a human or animal patient which is suffering from or susceptible to hepatic impairment or a condition that would be aggravated by hepatotoxicity, as defined above.

Also provided is a method of treating a human or animal patient suffering from or susceptible to a pathological condition or disease as defined above, which method comprises simultaneously, separately or sequentially administering to said human or animal patient a therapeutically effective amount of (a) methotrexate and (b) a DHODH inhibitor as defined above. Preferably in said method, (a) methotrexate and (b) the DHODH inhibitor are the sole active components.

Also provided is a method of treating a human or animal patient suffering from or susceptible to a pathological condition or disease as defined above, wherein the human or animal patient is suffering from or susceptible to hepatic impairment or a condition that would be aggravated by hepatotoxicity as defined above, which method comprises administering to said human or animal patient a therapeutically effective amount of a DHODH inhibitor as defined above.

Also provided is a combination as defined above for use in treating a pathological condition or disease as defined above.

Also provided is a DHODH inhibitor as defined above for use in treating a human or animal patient suffering from or susceptible to a pathological condition or disease as defined above, wherein the human or animal patient is suffering from or susceptible to hepatic impairment or a condition that would be aggravated by hepatotoxicity, as defined above.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination may be administered together in the same pharmaceutical composition or in different compositions intended for separate, simultaneous, concomitant or sequential administration by the same or a different route.

The combinations of the invention may conveniently be presented in unit dosage form and may be prepared by any of the methods well known in the art of pharmacy.

Combinations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the combination is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the combination is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

The combination may be in the form of a dry powder composition for topical delivery to the lung by inhalation. Dry powder compositions may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Genuair^{®} (formerly known as Novolizer SD2FL) which is described in the following patent applications: WO 97/000703, WO 03/000325 and WO 03/061742.

The combination may be in the form of a composition for nasal delivery. Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the combination is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Typically all active agents in the combination are administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

Preferably the drug combination of the invention is for administration as a dosage regime which involves administration of (i) 0.015 to 3 mg/kg/week of methotrexate, more preferably 0.07 to 0.7 mg/kg/week of methotrexate and most preferably 0.15 to 0.35 mg/kg/week of methotrexate, and (ii) 0.03 to 30 mg/kg/day of DHODH inhibitor, more preferably 0.07 to 14 mg/day of DHODH inhibitor and most preferably 0.15 to 0.3 mg/kg/day of DHODH inhibitor.

### EXAMPLES

### Example 1 - inhibition of human DHODH activity assay

DHODH activity and its inhibition were studied using a chromogen reduction assay with DCIP (2,6-dichlorophenol-indophenol). The substrate oxidation (Dihydroorotate, L-DHO), as well as co-substrate reduction (coenzyme Q, CoQ) is coupled to the chromogen reduction, hence enzymatic activity results in a loss of chromogen absorbance at 600 nm.

Enzyme extracts (8 µl, ∼1.5 µg of human protein) were incubated in 96-well plates. The assay mixture (200 µl) contained 200 µM CoQD, 100 µM L-DHO, 120 µM DCIP in the assay buffer (100 mM HEPES pH 8.0, 150 mM NaCl, 10% Glicerol, 0.05% Triton X-100) and 2 µl of test compound. The compounds were dissolved in DMSO at a stock concentration of 1 mM, and tested at different concentrations varying from 10 µM to 1 pM to calculate an IC₅₀(concentration of inhibitor required for 50% of inhibition).

The reaction was initiated by adding the enzyme and then incubated for 10 min at room temperature before measuring DCIP reduction by counting a decrease in absorbance at 600 nm using standard instrumentation (Spectramax).

All reactions were carried out in duplicate and graphs, determining IC₅₀ values for each compound, were plotted using the ABase software.

Table 1 shows the activities in human DHODH inhibition assay of some compounds of the present invention showing that these compounds are potent DHODH inhibitors.

**TABLE 1**

| **Compound** | **hDHODH IC₅₀ (nM)** |
|---|---|
| 1 | 105 |
| 4 | 98 |
| 6 | 57 |
| 7 | 49 |
| 8 | 18 |
| 14 | 57 |
| 15 | 113 |
| 18 | 62 |
| 19 | 10 |
| 22 | 114 |
| 25 | 28 |
| 30 | 41 |
| 31 | 119 |
| 34 | 109 |
| 41 | 190 |
| 42 | 30 |
| 44 | 78 |
| 50 | 138 |
| 51 | 21 |
| 54 | 19 |
| 57 | 91 |
| 60 | 53 |
| 66 | 28 |
| 67 | 11 |
| 71 | 14 |
| 73 | 190 |
| 75 | 97 |
| 77 | 12 |
| 79 | 33 |
| 85 | 32 |
| 89 | 5 |
| 91 | 6 |
| 93 | 20 |
| 95 | 10 |
| 97 | 5 |
| 101 | 37 |
| 105 | 2 |
| 107 | 7 |
| 110 | 145 |
| 112 | 4 |
| 116 | 90 |
| 119 | 19 |
| 121 | 3 |
| 123 | 57 |
| 125 | 9 |
| 126 | 12 |
| 127 | 10 |
| 128 | 9 |
| 129 | 12 |
| 131 | 38 |
| 135 | 21 |
| 138 | 8 |
| 141 | 146 |
| 144 | 77 |
| 148 | 46 |

### Example 2 - reduced hepatotoxicity

Acute hepatotoxicity assays were performed in Swiss mice. Animals received a single administration of either vehicle, or 100 mg/kg ofteriflunomide or a compound of the present invention by intraperitoneal route. Twenty-four hours later, animals were sacrificed and the levels of liver markers AST(aspartate aminotransferase), ALT(alanine aminotransferase) and BIL(total bilirubin) in plasma were determined.

**Table 2: Plasma levels of liver markers of mice after administration of 100 mg/kg of the compound, 100 mg/kg Teriflunomide or vehicle (IU: International Units).**

| **Compound** | ALT (IU/l) | AST (IU/l) | BIL (mg/dl) |
|---|---|---|---|
| **60** | 70 | 131 | 0.09 |
| **71** | 72 | 95 | 0.05 |
| **85** | 43 | 83 | 0.13 |
| **97** | 55 | 92 | 0.11 |
| **119** | 69 | 96 | 0.08 |
| **121** | 75 | 105 | 0.05 |
| **123** | 89 | 113 | 0.06 |
| **127** | 56 | 72 | 0.07 |
| **Vehicle** | 84 | 115 | 0.1 |
| **Teriflunomide** | 423 | 542 | 0.5 |

As it can clearly seen from Table 2, Teriflunomide-treated mice showed a dramatic increase in the three liver markers compared to vehicle-treated mice, clearly indicating a high hepatotoxicity, whereas the DHODH inhibitors disclosed in the present invention did not cause a significant increase in any of the parameters measured

### Example 3: Efficacy assay in adjuvant-induced arthritis of the combination product of the present invention.

The effect of DHODH inhibitor compounds were tested in combination with methotrexate (0.05 mg/Kg/ day) in the rat adjuvant-induced arthritis model (AIA) in animals with established disease (curative protocol). Briefly, Complete Freund Adjuvant (CFA) was injected into the left hind footpad of Wistar rats, and 10 days later the swelling of the two rear paws was measured with a plethysmometer. Rats exhibiting a similar degree of inflammation in both paws were randomized into treatment groups (n=7 per group). Compounds were administered orally once a day for 10 days and paw volumes were determined every two days up to day 21.

### Table 3. Effects of compound 20 (10 mg/Kg/day), Methotrexate(0.05 mg/Kg/day) and their combination on the inhibition of paw inflammation in arthritic rats.

Results are expressed as the mean±SEM inhibition of the inflammation measured as the area under the curve (AUC) of the right paw volumes in the period comprised between days 10 and 21 post-induction. The percentage of inhibition for every group was calculated versus values from vehicle-treated rats.

| **Treatment** | **% inhibition of the inflammation** **(AUC)** **Right paw** |
|---|---|
| Compound 20 (10mg/Kg) | 40±3 |
| MTX (0.05 mg/Kg) | 31±7 |
| Compound 20 (10 mg/Kg) + MTX (0.05 mg/Kg) | 56±6 |

Results from Table 3 show that Compound 20 inhibits the inflammation caused by experimental arthritis in rats. Furthermore, the co-administration of MTX and compound 20 resulted in an increased efficacy (**35 %**) versus compound A alone, thus indicating the feasibility of administering the compound in patients treated with MTX.

## Claims

1. A combination which comprises (a) methotrexate and (b) a non-hepatotoxic DHODH inhibitor of formula (I): 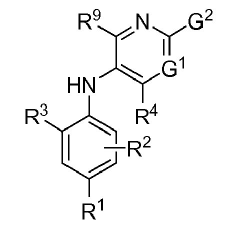 wherein:
R¹ is selected from the group consisting of hydrogen atoms, halogen atoms, C₁₋₄ alkyl, C₃₋₄ cycloalkyl, -CF₃ and -OCF₃,
R² is selected from the group consisting of hydrogen atoms, halogen atoms and C₁₋₄ alkyl groups,
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ represents a group selected from N and CR⁶ wherein R⁶ is selected from the group consisting of hydrogen atoms, halogen atoms, C₁₋₄ alkyl, C₃₋₄ cycloalkyl, C₁₋₄ alkoxy, - CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G² represents a group selected from:
• a hydrogen atom, a hydroxy group, a halogen atom, a C₃₋₄ cycloalkyl group, a C₁₋₄ alkoxy group and -NR^{a}R^{b}, wherein R^{a} represents a C₁₋₄ alkyl group and R^{b} is selected from a group consisting of C₁₋₄ alkyl group and C₁₋₄ alkoxy-C₁₋₄ alkyl group, or R^{a} and R^{b} together with the nitrogen atom to which they are attached form a saturated 6 to 8 membered heterocyclic ring optionally containing one oxygen atom as an additional heteroatom,
• a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 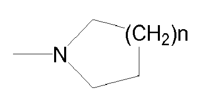 wherein n is an integer from 0 to 3,
and
• a phenyl group which is optionally substituted by one or more substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 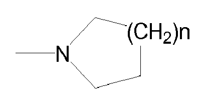
wherein n is an integer from 0 to 3
or, when G' represents CR⁶, G² together with R⁶ forms a non-aromatic C₅₋₁₀ carbocyclic group or a C₆₋₁₀ aryl group, and the pharmaceutically acceptable salts and N-oxides thereof.

2. A combination according to claim 1, wherein R¹ is selected from the group consisting of hydrogen atoms, fluorine atoms, chlorine atoms, bromine atoms, C₁₋₄ alkyl, C₃₋₄ cycloalkyl, and -CF₃ groups.

3. A combination according to claim 1 or 2, wherein R² is selected from the group consisting of hydrogen atoms, halogen atoms and a methyl group.

4. A combination according to any one of claims 1 to 3, claims wherein G¹ is selected from the group consisting of nitrogen atoms, CCI, CF, CH, C(CH₃), C(cyclopropyl), C(phenyl) and C(CF₃) groups.

5. A combination according to any one of claims 1 to 4, wherein G² represents a group selected from:
• a hydrogen atom, a halogen atom, a C₃₋₄ cycloalkyl group, a C₁₋₂ alkoxy group and - NR^{a}R^{b}, wherein R^{a} represents a C₁₋₂ alkyl group and R^{b} is selected from the group consisting of C₁₋₂ alkyl groups and C₁₋₂ alkoxy-C₁₋₂ alkyl groups, or R^{a} and R^{b} together with the nitrogen atom to which they are attached form a saturated 6 or 7 membered heterocyclic ring optionally containing one oxygen atom as an additional heteroatom,
• a monocyclic or bicyclic 5 to 10 membered heteroaromatic ring containing one or two nitrogen atoms which is optionally substituted by one or more substituents selected from halogen atoms and C₁₋₄ alkyl groups,
and
• a phenyl group which is optionally substituted by one, two or three substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸ and oxadiazolyl groups, which oxadiazolyl groups are optionally substituted by a C₁₋₄ alkyl or a C₃₋₇ cycloalkyl group and wherein R⁷ and R⁸ are independently selected from hydrogen atoms, linear or branched C₁₋₄ alkyl groups, C₃₋₄ cycloalkyl groups, or R⁷ and R⁸ together with the nitrogen atom to which they are attached form a group of formula 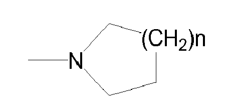
wherein n is 1 or 2,
or, when G' represents CR⁶, G² together with R⁶ forms a non-aromatic C₆ carbocyclic group or a phenyl group.

6. A combination according to any one of claims 1 to 5, wherein G² represents a group selected from:
• a hydrogen atom, a fluorine atom, a cyclopropyl group, a methoxy group, -NMeEt, -NEt₂, -N(Me)-(CH₂)₂-O-CH₃, 6-morpholinyl, azepan-1-yl and piperidin-1-yl,
• a pyridinyl, pyrimidinyl, quinolinyl or pyrazinyl ring optionally substituted with one or two substituents selected from Me and F
and
• a phenyl group which is optionally substituted by one, two or three substituents selected from fluorine, chlorine, methyl, hydroxyl, methoxy, ethoxy, isopropyloxy, cyclopropyl, cyclopropyloxy, cyano, -CF₃, -OCF₃ ,oxadiazolyl and -CONR⁷R⁸ groups, which oxadiazolyl groups are optionally substituted by a methyl group and wherein R⁷ and R⁸ are independently selected from hydrogen atom, methyl group, isopropyl group, cyclopropyl group, or R⁷ and R⁸ together with the nitrogen atom to which they are attached form a group of formula 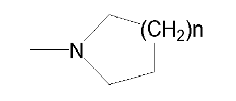 wherein n is 1,
or, when G' represents CR⁶, G² together with R⁶ forms a non-aromatic C₆ carbocyclic group or a phenyl group.

7. A combination according to any one of claims 1 to 6, wherein G² represents a group selected from methoxy group, cyclopropyl group and optionally substituted phenyl, pyridyl, quinolynyl, pyrimidinyl and pyrazinyl groups.

8. A combination according to claim 1, wherein:
R⁹ represents a hydrogen atom, and
G² represents a group selected from:
• a monocyclic or bicyclic 5 to 10 membered heteroaromatic ring containing a nitrogen atom which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 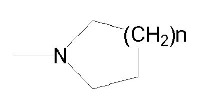 wherein n is an integer from 0 to 3,
and
• a phenyl group which is optionally substituted by one or more substituents selected from halogen atoms, C₁₋₄ alkyl, C₁₋₄ alkoxy, C₃₋₄cycloalkyl, C₃₋₄ cycloalkoxy, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R⁸ are independently selected from hydrogen atoms, linear or branched C₁₋₄ alkyl groups, C₃₋₇ cycloalkyl groups, or R⁷ and R⁸ together with the nitrogen atom to which they are attached form a group of formula 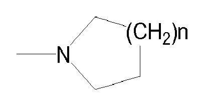
wherein n is an integer from 0 to 3.

9. A combination according to any one of claims 1 to 8, wherein R¹ is selected from the group consisting of C₁₋₄ alkyl, C₃₋₄ cycloalkyl and -CF₃ groups, typically selected from the group consisting of methyl and cyclopropyl groups, preferably a cyclopropyl group.

10. A combination according to any one of claims 1 to 9, wherein R² is selected from a hydrogen or halogen atom, typically a hydrogen atom.

11. A combination according to any one of claims 1 to 10, wherein R³ is selected from the group consisting of COOR⁵, -CONHR⁵ and tetrazolyl groups, typically a -COOH group.

12. A combination according to any one of claims 1 to 11, wherein R⁴ represents a hydrogen atom or a methyl group, typically a hydrogen atom.

13. A combination according to any one of claims 1 to 12, wherein R⁹ represents a hydrogen atom.

14. A combination according to any one of claims 1 to 13, wherein G¹ is selected from the group consisting of nitrogen atoms and CH, C(CH₃), C(cyclopropyl), C(phenyl) and C(CF₃) groups.

15. A combination according to any one of claims 1 to 14, wherein G² represents a group selected from optionally substituted phenyl, pyridyl, quinolynyl, pyrimidinyl and pyrazinyl groups, typically selected from the group consisting of an optionally substituted phenyl, 4-pyridyl, 5-quinolynyl and 2-pyrazinyl groups.

16. A combination according to claim 1, wherein R¹ is selected from a methyl or cyclopropyl group, R² represents a hydrogen atom, R³ is a COOH group, R⁴ represents a hydrogen atom or a methyl group, G¹ is selected from nitrogen atoms and CH, C(CH₃), C(cyclopropyl), C(phenyl) and C(CF₃) groups and G² represents a group selected from the group consisting of an optionally substituted phenyl, 4-pyridyl, 5-quinolynyl and 2-pyrazinyl groups.

17. A combination according to claim 16, wherein R⁹ represents a hydrogen atom.

18. A combination according to claim 1, wherein R¹ is selected from a methyl or cyclopropyl group, R² represents a hydrogen atom, R³ is a COOH group, R⁴ represents a hydrogen atom, G¹ is selected from nitrogen atoms and CH, C(CH₃) and C(CF₃) groups and G² represents a phenyl group optionally substituted with one or two substituents selected from chloro, fluoro, methoxy, ethoxy, isopropoxy, trifluoromethoxy and -CONR⁷R⁸, wherein R⁷ is hydrogen and R⁸ is cyclopropyl or R⁷ and R⁸ together with the nitrogen atom to which they are attached form a group of formula 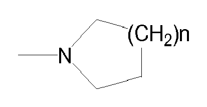 wherein n is 1.

19. A combination according to claim 1, wherein the DHODH inhibitor is one of:
5-cyclopropyl-2-(2-phenylpyrimidin-5-ylamino)benzoic acid
2-(6-Cyclopropyl-5-methylpyridin-3-ylamino)-5-methylbenzoic acid
5-(2-Carboxy-4-cyclopropylphenylamino)-3-methyl-2-phenylpyridine 1-oxide
5-Methyl-2-(6-(3-(trifluoromethyl)phenyl)pyridin-3-ylamino)benzoic acid
5-cyclopropyl-2-(6-hydroxy-5-phenylpyridin-3-ylamino)benzoic acid
5-cyclopropyl-2-(2-(2,6-difluoro-4-hydroxyphenyl)pyrimidin-5-ylamino)benzoic acid
5-Cyclopropyl-2-(6-methoxy-5-phenylpyridin-3-ylamino) benzoic acid
2-(5-Fluoro-6-phenylpyridin-3-ylamino)-5-methylbenzoic acid
2-(6-(Ethyl(methyl)amino)-5-methylpyridin-3-ylamino)-5-methylbenzoic acid
5-Cyclopropyl-2-(3'-fluoro-2,4'-bipyridin-5-ylamino)benzoic acid
2-(6-(Diethylamino)-5-methylpyridin-3-ylamino)-5-methylbenzoic acid
2-(6-((2-Methoxyethyl)(methyl)amino)-5-methylpyridin-3-ylamino)-5-methylbenzoic acid
2-(5-Chloro-6-phenylpyridin-3-ylamino)-5-methylbenzoic acid
5-Cyclopropyl-2-(2-(2-cyclopropylphenyl)pyrimidin-5-ylamino)benzoic acid
5-cyclopropyl-2-(5-phenylpyridin-3-ylamino) benzoic acid
5-methyl-2-(quinolin-3-ylamino)benzoic acid
5-methyl-2-(5,6,7,8-tetrahydroquinolin-3-ylamino)benzoic acid
2-(5-Chloro-2-phenylpyridin-3-ylamino)-5-methylbenzoic acid
5-Cyclopropyl-2-(5,6-diphenylpyridin-3-ylamino)benzoic acid
5-cyclopropyl-2-(2-(2,6-difluorophenyl)pyrimidin-5-ylamino)benzoic acid
5-Cyclopropyl-2-(5-methylpyridin-3-ylamino) benzoic acid
2-(2-(3-Cyclopropoxyphenyl)pyrimidin-5-ylamino)-5-cyclopropylbenzoic acid
5-Methyl-2-(6-morpholinopyridin-3-ylamino)benzoic acid
5-Methyl-2-(5-methyl-6-morpholinopyridin-3-ylamino)benzoic acid
5-cyclopropyl-2-(6-cyclopropyl-5-phenylpyridin-3-ylamino) benzoic acid
2-(6-(2-Cyclopropylphenyl)-5-methylpyridin-3-ylamino)-5-methylbenzoic acid
2-(6-(2-Cyanophenyl)-5-methylpyridin-3-ylamino)-5-methylbenzoic acid
2-(2-(3-Chlorophenyl)pyrimidin-5-ylamino)-5-cyclopropylbenzoic acid
5-Methyl-2-(6-phenyl-5-(trifluoromethyl)pyridin-3-ylamino)benzoic acid
5-Methyl-2-(5-methyl-6-(piperidin-1-yl)pyridin-3-ylamino)benzoic acid
2-(6-(Azepan-1-yl)-5-methylpyridin-3-ylamino)-5-methylbenzoic acid
2-(6-(3-Methoxyphenyl)-5-phenylpyridin-3-ylamino)-5-methylbenzoic acid
2-(2,3'-bipyridin-5-ylamino)-5-cyclopropylbenzoic acid
2-(3'-chloro-2,4'-bipyridin-5-ylamino)-5-methylbenzoic acid
5-Methyl-2-(3-methyl-2,2'-bipyridin-5-ylamino)benzoic acid
2-(5,6-Difluoropyridin-3-ylamino)-5-methylbenzoic acid
2-(6-(3-Methoxyphenyl)pyridin-3-ylamino)benzoic acid
2-(6-(3-Ethoxyphenyl)pyridin-3-ylamino)benzoic acid
2-(6-(3-Ethoxyphenyl)pyridin-3-ylamino)-5-fluorobenzoic acid
2-(6-(3-Ethoxyphenyl)-5-methylpyridin-3-ylamino)benzoic acid
2-(6-(3-Ethoxyphenyl)pyridin-3-ylamino)-5-methylbenzoic acid
2-(6-(3-Ethoxyphenyl)-5-methylpyridin-3-ylamino)-5-methylbenzoic acid
2-(6-(3-Ethoxy-2-fluorophenyl)pyridin-3-ylamino)benzoic acid
2-(6-(3-Ethoxyphenyl)-4-methylpyridin-3-ylamino)-5-methylbenzoic acid
2-(6-(3-Ethoxyphenyl)-4-methylpyridin-3-ylamino)benzoic acid
5-Bromo-2-(6-(3-ethoxyphenyl)pyridin-3-ylamino)benzoic acid
5-Chloro-2-(6-(3-ethoxyphenyl)pyridin-3-ylamino)benzoic acid
2-(6-(5-Ethoxy-2-ffuorophenyl)pyridin-3-ylamino)benzoic acid
2-(6-(3-Ethoxyphenyl)-5-methylpyridin-3-ylamino)-5-(trifluoromethyl)benzoic acid
2-(6-(3-Methoxyphenyl)-5-methylpyridin-3-ylamino)-5-(trifluoromethyl)benzoic acid
2-(6-(3-Methoxyphenyl)-5-methylpyridin-3-ylamino)-5-methylbenzoic acid
2-(6-(3-Methoxyphenyl)-5-methylpyridin-3-ylamino)-6-methylbenzoic acid
5-Fluoro-2-(6-(3-methoxyphenyl)-5-methylpyridin-3-ylamino)benzoic acid
2-(6-(5-Ethoxy-2-ffuorophenyl)pyridin-3-ylamino)-5-methylbenzoic acid
2-(6-(2-Fluoro-5-methoxyphenyl)-5-methylpyridin-3-ylamino)-5-methylbenzoic acid Ethyl 2-(6-(2-fluoro-5-methoxyphenyl)-5-methylpyridin-3-ylamino)-5-methylbenzoate
2-(6-(2-Fluorophenyl)pyridin-3-ylamino)-5-methylbenzoic acid
2-(6-(3-Methoxyphenyl)-5-phenylpyridin-3-ylamino)-5-methylbenzoic acid Ethyl 2-(6-(3-methoxyphenyl)-5-phenylpyridin-3-ylamino)-5-methylbenzoate
5-Methyl-2-(5-methyl-6-phenylpyridin-3-ylamino)benzoic acid Ethyl 5-methyl-2-(5-methyl-6-phenylpyridin-3-ylamino)benzoate
5-Methyl-2-(5-methyl-6-(3-(trifluoromethoxy)phenyl)pyridin-3-ylamino)benzoic acid Ethyl 5-methyl-2-(5-methyl-6-(3-(trifluoromethoxy)phenyl)pyridin-3-ylamino)benzoate
2-(5-Cyclopropyl-6-(3-methoxyphenyl)pyridin-3-ylamino)-5-methylbenzoic acid Ethyl 2-(5-cyclopropyl-6-(3-methoxyphenyl)pyridin-3-ylamino)-5-methylbenzoate
2-(6-(2-Fluoro-5-isopropoxyphenyl)pyridin-3-ylamino)-5-methylbenzoic acid
2-(6-(3-Isopropoxyphenyl)-5-methylpyridin-3-ylamino)-5-methylbenzoic acid Ethyl 2-(6-(3-isopropoxyphenyl)-5-methylpyridin-3-ylamino)-5-methylbenzoate
2-(6-(3-Cyclopropoxyphenyl)-5-methylpyridin-3-ylamino)-5-methylbenzoic acid *tert*-Butyl 2-(6-(3-cyclopropoxyphenyl)-5-methylpyridin-3-ylamino)-5-methylbenzoate
2-(6-(2-Chlorophenyl)-5-methylpyridin-3-ylamino)-5-methylbenzoic acid *tert*-Butyl 2-(6-(2-chlorophenyl)-5-methylpyridin-3-ylamino)-5-methylbenzoate
2-(6-(3-Carbamoylphenyl)-5-methylpyridin-3-ylamino)-5-methylbenzoic acid Ethyl 2-(6-(3-carbamoylphenyl)-5-methylpyridin-3-ylamino)-5-methylbenzoate
2-(6-(2-Fluoro-5-methoxyphenyl)-4-methylpyridin-3-ylamino)-5-methylbenzoic acid Ethyl 2-(6-(2-fluoro-5-methoxyphenyl)-4-methylpyridin-3-ylamino)-5-methylbenzoate
2-(6-(3-Methoxyphenyl)-5-(trifluoromethyl)pyridin-3-ylamino)-5-methylbenzoic acid Ethyl 2-(6-(3-methoxyphenyl)-5-(trifluoromethyl)pyridin-3-ylamino)-5-methylbenzoate
2-(6-(3-(Dimethylcarbamoyl)phenyl)-5-methylpyridin-3-ylamino)-5-methylbenzoic acid Ethyl 2-(6-(3-(dimethylcarbamoyl)phenyl)-5-methylpyridin-3-ylamino)-5-methylbenzoate
2-(6-(3-Isopropoxyphenyl)-5-methylpyridin-3-ylamino)-3-methylbenzoic acid *tert-Butyl* 2-(6-(3-isopropoxyphenyl)-5-methylpyridin-3-ylamino)-3-methylbenzoate
3-Methyl-2-(5-methyl-6-phenylpyridin-3-ylamino)benzoic acid *tert-Butyl* 3-methyl-2-(5-methyl-6-phenylpyridin-3-ylamino)benzoate
2-(6-(2-Chlorophenyl)pyridin-3-ylamino)-5-methylbenzoic acid *tert*-Butyl 2-(6-(2-chlorophenyl)pyridin-3-ylamino)-5-methylbenzoate
3-Fluoro-2-(6-(3-methoxyphenyl)-5-methylpyridin-3-ylamino)benzoic acid *tert*-Butyl 3-fluoro-2-(6-(3-methoxyphenyl)-5-methylpyridin-3-ylamino) benzoate
5-Cyclopropyl-2-(5-methyl-6-(3-(trifluoromethoxy)phenyl)pyridin-3-ylamino)benzoic acid Ethyl 5-cyclopropyl-2-(5-methyl-6-(3-(trifluoromethoxy)phenyl)pyridin-3-ylamino)benzoate
5-Cyclopropyl-2-(5-methyl-6-phenylpyridin-3-ylamino)benzoic acid Ethyl 5-cyclopropyl-2-(5-methyl-6-phenylpyridin-3-ylamino)benzoate
5-Methyl-2-(5-methyl-6-(2-(trifluoromethyl)phenyl)pyridin-3-ylamino)benzoic acid *tert*-Butyl 5-methyl-2-(5-methyl-6-(2-(trifluoromethyl)phenyl)pyridin-3-ylamino)benzoate
2-(6-(3-Chlorophenyl)-5-methylpyridin-3-ylamino)-5-methylbenzoic acid *tert*-Butyl 2-(6-(3-chlorophenyl)-5-methylpyridin-3-ylamino)-5-methylbenzoate
2-(6-(2-Fluorophenyl)-5-methylpyridin-3-ylamino)-5-methylbenzoic acid *tert-Butyl* 2-(6-(2-fluorophenyl)-5-methylpyridin-3-ylamino)-5-methylbenzoate
5-Methyl-2-(5-methyl-6-(quinolin-5-yl)pyridin-3-ylamino)benzoic acid *tert-Butyl* 5-methyl-2-(5-methyl-6-(quinolin-5-yl)pyridin-3-ylamino)benzoate
2-(3'-Fluoro-3-methyl-2,4'-bipyridin-5-ylamino)-5-methylbenzoic acid *tert-Butyl 2-(3'-fluoro-3-methyl-2,4'-bipyridin-5-ylamino)-5-methylbenzoate*
5-Methyl-2-(5-methyl-6-(pyrazin-2-yl)pyridin-3-ylamino)benzoic acid *tert*-Butyl 5-methyl-2-(5-methyl-6-(pyrazin-2-yl)pyridin-3-ylamino)benzoate
5-Cyclopropyl-2-(6-phenyl-5-(trifluoromethyl)pyridin-3-ylamino)benzoic acid Ethyl 5-cyclopropyl-2-(6-phenyl-5-(trifluoromethyl)pyridin-3-ylamino)benzoate
5-Cyclopropyl-2-(6-(3-methoxyphenyl)-5-(trifluoromethyl)pyridin-3-ylamino)benzoic acid Ethyl 5-cyclopropyl-2-(6-(3-methoxyphenyl)-5-(trifluoromethyl)pyridin-3-ylamino)benzoate
5-Chloro-2-(6-(2-fluorophenyl)pyridin-3-ylamino)benzoic acid
5-Chloro-2-(6-(2-chlorophenyl)pyridin-3-ylamino)benzoic acid
5-Chloro-2-(6-(quinolin-5-yl)pyridin-3-ylamino)benzoic acid
2-(6-(2-Chlorophenyl)pyridin-3-ylamino)-5-cyclopropylbenzoic acid Ethyl 2-(6-(2-chlorophenyl)pyridin-3-ylamino)-5-cyclopropylbenzoate
5-Chloro-2-(6-(2-(trifluoromethyl)phenyl)pyridin-3-ylamino)benzoic acid
5-Fluoro-2-(6-(2-(trifluoromethyl)phenyl)pyridin-3-ylamino)benzoic acid
2-(3'-Fluoro-2,4'-bipyridin-5-ylamino)-5-methylbenzoic acid
2-(2-(2-Fluorophenyl)pyrimidin-5-ylamino)-5-methylbenzoic acid tert-Butyl 2-(2-(2-fluorophenyl)pyrimidin-5-ylamino)-5-methylbenzoate
2-(6-(2,6-Difluorophenyl)pyridin-3-ylamino)-5-methylbenzoic acid Ethyl 2-(6-(2,6-difluorophenyl)pyridin-3-ylamino)-5-methylbenzoate
2-(2-(2-Chlorophenyl)pyrimidin-5-ylamino)-5-cyclopropylbenzoic acid Methyl 2-(2-(2-chlorophenyl)pyrimidin-5-ylamino)-5-cyclopropylbenzoate
2-(2-(2-Chlorophenyl)pyrimidin-5-ylamino)-5-methylbenzoic acid *tert*-Butyl 2-(2-(2-chlorophenyl)pyrimidin-5-ylamino)-5-methylbenzoate
5-Methyl-2-(5-methyl-6-(3-(pyrrolidine-1-carbonyl)phenyl)pyridin-3-ylamino)benzoic acid
2-(6-(3-(Cyclopropylcarbamoyl)phenyl)-5-methylpyridin-3-ylamino)-5-methylbenzoic acid
5-Cyclopropyl-2-(2-(2-fluorophenyl)pyrimidin-5-ylamino)benzoic acid
2-(2-(2-trifluoromethylphenyl)pyrimidin-5-ylamino)-5-cyclopropylbenzoic acid
2-(2-o-tolylpyrimidin-5-ylamino)-5-cyclopropylbenzoic acid
2-(2-(2-cyclopropoxyphenyl)pyrimidin-5-ylamino)-5-cyclopropylbenzoic acid
2-(2-(2,5-difluorophenyl)pyrimidin-5-ylamino)-5-cyclopropylbenzoic acid
2-(2-(2,3-difluorophenyl)pyrimidin-5-ylamino)-5-cyclopropylbenzoic acid
2-(2-(2-fluoro-5-chlorophenyl)pyrimidin-5-ylamino)-5-cyclopropylbenzoic acid
2-(2-(2-trifluoromethylphenyl)pyrimidin-5-ylamino)-5-methylbenzoic acid
2-(2-(2-fluoro-5-trifluoromethoxyphenyl)pyrimidin-5-ylamino)-5-cyclopropylbenzoic acid
2-(6-(2-trifluoromethylphenyl)pyridin-3-ylamino)-5-methylbenzoic acid
2-(6-phenylpyridin-3-ylamino)-5-cyclopropylbenzoic acid
2-(6-(2-fluorophenyl)pyridin-3-ylamino)-5-cyclopropylbenzoic acid
2-(6-(3,5-difluoropyridin-4-yl)pyridin-3-ylamino)-5-methylbenzoic acid
2-(6-(3-cyclopropylcarbamoylphenyl)pyridin-3-ylamino)-5-methylbenzoic acid
2-(6-(2,4-difluorophenyl)pyridin-3-ylamino)-5-methylbenzoic acid
2-(6-(2,5-difluorophenyl)pyridin-3-ylamino)-5-methylbenzoic acid
2-(6-(2-fluorophenyl)pyridin-3-ylamino)-5-cyclopropyl-3-fluorobenzoic acid
2-(6-(2,3,6-trifluorophenyl)pyridin-3-ylamino)-5-methylbenzoic acid
2-(6-(3-(5-methyl-1,3,4-oxadiazol-2-yl)phenyl)pyridin-3-ylamino)-5-methylbenzoic acid
2-(5-methyl-6-(pyrimidin-5-yl)pyridin-3-ylamino)-5-methylbenzoic acid
2-(6-(2,3-difluorophenyl)pyridin-3-ylamino)-5-methylbenzoic acid
2-(6-(5-fluoro-2-methoxyphenyl)-5-methylpyridin-3-ylamino)-5-methylbenzoic acid
2-(6-(4-carbamoylphenyl)-5-methylpyridin-3-ylamino)-5-methylbenzoic acid
or a pharmaceutically acceptable salt or N-oxide thereof.

20. A combination according to claim 1 wherein the DHODH inhibitor is 5-Methyl-2-(6-(3-(trifluoromethyl)phenyl)pyridin-3-ylamino)benzoic acid or a pharmaceutically acceptable salt or N-oxide thereof

21. A combination according to claim 1 wherein the DHODH inhibitor is 5-cyclopropyl-2-(2-(2,6-difluorophenyl)pyrimidin-5-ylamino)benzoic acid or a pharmaceutically acceptable salt or N-oxide thereof

22. A combination according to claim 1 wherein the DHODH inhibitor is 2-(6-(2,6-difluorophenyl)pyridin-3-ylamino)-5-methylbenzoic acid or a pharmaceutically acceptable salt or N-oxide thereof

23. A combination according to claim 1 wherein the DHODH inhibitor is 2-(6-(3-(cyclopropylcarbamoyl)phenyl)-5-methylpyridin-3-ylamino)-5-methylbenzoic acid or a pharmaceutically acceptable salt or N-oxide thereof

24. A combination according to any one of the preceding claims **characterised in that** the active ingredients (a) and (b) form part of a single pharmaceutical composition.

25. A combination according to any one of the preceding claims, which further comprises (c) another compound selected from:
(i) Anti-TNF-alpha monoclonal antibodies such as Infliximab, Certolizumab pegol, Golimumab, Adalimumab and AME-527 from Applied Molecular Evolution
(ii) TNF-alpha Antagonists such as Etanercept, Lenercept, Onercept and Pegsunercept
(iii) Calcineurin (PP-2B) Inhibitors / INS Expression Inhibitors such as cyclosporine A, Tacrolimus and ISA-247 from Isotechnika
(iv) IL-1 Receptor Antagonists such as Anakinra and AMG-719 from Amgen
(v) Anti-CD20 monoclonal antibodies such as Rituximab, Ofatumumab, Ocrelizumab and TRU-015 from Trubion Pharmaceuticals
(vi) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745 and VX-702 (all from Vertex)
(vii) NF-kappaB (NFKB) Activation Inhibitors such as Sulfasalazine and Iguratimod
(viii) Another dihydrofolate reductase (DHFR) inhibitor such as Aminopterin and CH-1504 from Chelsea.
(ix) Janus kinase (JAK) inhibitors, such as CP-690, 550 from Pfizer and INCB-18424, from Incyte.
(x) MEK inhibitor, such as ARRY-162 from Array, and
(xi) Sphingosine-1 phosphate receptor agonists, such as fingolimod (Novartis)

26. Use of (a) methotrexate and (b) a DHODH inhibitor as defined in any one claims 1 to 23 for the preparation of a medicament for simultaneous, separate or sequential use in the treatment of a pathological condition or disease susceptible to amelioration by inhibition of dehydroorotate dehydrogenase.

27. Use according to claim 26 wherein the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis.

28. A product comprising (a) methotrexate and (b) a DHODH inhibitor as defined in any one of claims 1 to 24, as a combined preparation for simultaneous, separate or sequential use in the treatment of a human or animal patient suffering from or susceptible to a pathological condition or disease as defined in claim 26 or 27.

29. A product according to claim 28, which further comprises an active compound (c), as defined in claim 25.

30. A kit of parts comprising (b) a DHODH inhibitor as defined in any one of claims 1 to 24 together with instructions for simultaneous, separate or sequential use in combination with (a) methotrexate for the treatment of a human or animal patient suffering from or susceptible to a pathological condition or disease as defined in claim 26 or 27.

31. A kit according to claim 30, which further comprises an active compound (c), as defined in claim 25.

32. A package comprising (b) a DHODH inhibitor as defined in any one of claims 1 to 24 and (a) methotrexate, for simultaneous, separate or sequential use in the treatment of a pathological condition or disease as defined in claim 26 or 27.

33. A package according to claim 32, which further comprises an active compound (c), as defined in claim 25.

34. Use of (b) a DHODH inhibitor as defined in any one of claims 1 to 24 for the preparation of a medicament, for use in combination with (a) methotrexate, for the treatment of a pathological condition or disease as defined in claim 26 or 27.

35. Use of (a) methotrexate, for the preparation of a medicament for use in combination with (b) a DHODH inhibitor as defined in any one of claims 1 to 24 for the treatment of a pathological condition or disease as defined in claim 26 or 27.

36. Use according to claim 26, 27, 34 or 35, wherein the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor.

37. Use according to any one of claims 26, 27 and 34 to 36, wherein the medicament is for use in treating a human or animal patient suffering from or susceptible to hepatic impairment or a condition that would be aggravated by hepatotoxicity.

38. Use according to claim 37, wherein said condition that would be aggravated by hepatotoxicity is liver fibrosis, hepatitis, cirrhosis or liver cancer.

39. Use of a DHODH inhibitor as defined in any one of claims 1 to 24 in the manufacture of a medicament for use in treating a human or animal patient suffering from or susceptible to a pathological condition or disease as defined in claim 26 or 27, wherein the human or animal patient is suffering from or susceptible to hepatic impairment or a condition that would be aggravated by hepatotoxicity, as defined in claim 37 or 38.

40. A method of treating a human or animal patient suffering from or susceptible to a pathological condition or disease as defined in claim 26 or 27, which method comprises simultaneously, separately or sequentially administering to said human or animal patient a therapeutically effective amount of (a) methotrexate and (b) a DHODH inhibitor as defined in any one of claims 1 to 24.

41. A method of treating a human or animal patient suffering from or susceptible to a pathological condition or disease as defined in claim 26 or 27, wherein the human or animal patient is suffering from or susceptible to hepatic impairment or a condition that would be aggravated by hepatotoxicity as defined in claim 37 or 38, which method comprises administering to said human or animal patient a therapeutically effective amount of a DHODH inhibitor as defined in any one of claims 1 to 24.

42. A combination according to any of claims 1 to 25 for use in treating a pathological condition or disease as defined in claim 26 or 27.

43. A DHODH inhibitor as defined in any one of claims 1 to 24 for use in treating a human or animal patient suffering from or susceptible to a pathological condition or disease as defined in claim 26 or 27, wherein the human or animal patient is suffering from or susceptible to hepatic impairment or a condition that would be aggravated by hepatotoxicity as defined in claim 37 or 38.
